# EUROPÄISCHE PATENTANMELDUNG

(11) **EP 4 382 109 A1**
(43) Veröffentlichungstag der Anmeldung: **12.06.2024**
(21) Anmeldenummer: 23209625.5
(22) Anmeldetag: 14.11.2023
(51) Int. Cl.: A61K 31/7036, A61L 27/54, A61P 19/00, A61P 19/08

(54) **ANWENDUNG EINES KOMPOSITMATERIALS**

(30) Priorität: 06.12.2022 EP 22211559; 26.10.2023 EP 23206010
(71) Anmelder: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Heraeus IP

(57) **Zusammenfassung**

Die Erfindung betrifft eine Zusammensetzung zur Anwendung in einem Verfahren zur Behandlung eines Knochendefekts, welche Calciumsulfat, ein Erdalkalicarbonat, ein Bindemittel, und bis zu 2,5 Gewichtsprozent Gentamicinsulfat, bezogen auf die Gesamtmasse der Zusammensetzung, umfasst, sowie ein Herstellungsverfahren für eine solche Zusammensetzung. Die Zusammensetzung kann als Knochenersatzmaterial verwendet werden.

## Beschreibung

### GEBIET DER ERFINDUNG

Die vorliegende Erfindung betrifft das Gebiet der Orthopädie, insbesondere Kompositmaterialien zur Anwendung als Knochenersatzmaterial.

### TECHNISCHER HINTERGRUND

Knochenersatzmaterialien können zum Auffüllen von Knochenkavitäten verwendet werden. Grundsätzlich kann dies mit patienteneigenem Gewebe erfolgen, welches für den Patienten gut verträglich ist, jedoch oft nur in begrenzter Menge gewonnen werden kann. Für größere Knochenkavitäten müssen daher in der Praxis häufig künstliche Materialien als Knochenersatzmaterialien verwendet werden. Calciumsulfat-haltige Knochenersatzmaterialien sind beispielsweise in den Patentdokumenten US2002110541A, US5807567A, US2002197315A, US6652887A, US5756127A, US5614206A und DE19953771C beschrieben. Bei chirurgischen Operationen besteht grundsätzlich die Gefahr, dass eine mikrobielle Infektion durch den medizinischen Eingriff erfolgt, beispielsweise durch mikrobiell belastete Knochenersatzmaterialien oder medizinische Instrumente. In anderen Fällen liegt bereits vor der Operation eine Infektion vor, und es besteht ein Risiko, dass die verursachenden Krankheitserreger während der Operation nicht vollständig von dem befallenen Gewebe entfernt werden können.

### BEVORZUGTE AUSFÜHRUNGSFORMEN

Die vorliegende Anmeldung beschreibt Knochenersatzmaterialien und deren Herstellung. Die Knochenersatzmaterialien können ein Antibiotikum enthalten, welches bevorzugt verzögert aus den Knochenersatzmaterialien freigesetzt wird. Weiterhin werden therapeutische Anwendungen von Knochenersatzmaterialien beschrieben.

Bevorzugte Ausführungsformen der Erfindung werden nachstehend beschrieben.

Ein erster Aspekt betrifft in einer ersten Ausführungsform eine Zusammensetzung zur Anwendung in einem Verfahren zur Behandlung eines Knochendefekts, wobei die Zusammensetzung Calciumsulfat, ein Erdalkalicarbonat, ein Bindemittel, und mehr als 0 bis höchstens 2,5 Gewichtsprozent Gentamicinsulfat, bevorzugt 0,5 bis 2,5 Gewichtsprozent Gentamicinsulfat, bezogen auf die Gesamtmasse der Zusammensetzung, umfasst.

In einer zweiten Ausführungsform umfasst die Anwendung ein Vermischen der Zusammensetzung mit lebensfähigen Zellen *in vitro,* wobei durch das Vermischen bevorzugt ein homogenes Gemisch gebildet wird.

In einer dritten Ausführungsform der Zusammensetzung zur Anwendung gemäß der zweiten Ausführungsform werden die lebensfähigen Zellen in Form von Blut, bevorzugt Vollblut, gereinigten Knochenmarkzellen, und/oder in Form von Spongiosa *in vitro* mit der Zusammensetzung vermischt.

In einer vierten Ausführungsform der Zusammensetzung zur Anwendung gemäß der dritten Ausführungsform sind die lebensfähigen Zellen autolog oder allogen.

In einer fünften Ausführungsform der Zusammensetzung zur Anwendung gemäß Ausführungsform 3 oder 4, liegt die Spongiosa in Form von Knochenchips vor.

In einer sechsten Ausführungsform der Zusammensetzung zur Anwendung gemäß der fünften Ausführungsform weisen die Knochenchips einen mittleren Durchmesser von 1 bis 10 mm auf.

In einer siebten Ausführungsform der Zusammensetzung zur Anwendung gemäß einer der Ausführungsformen 3 bis 6 wird die Zusammensetzung *in vitro* in einem Gewichtsverhältnis von "1 zu 0,5" bis "1 zu 3" mit Spongiosa oder gereinigten Knochenmarkzellen vermischt.

In einer achten Ausführungsform der Zusammensetzung zur Anwendung gemäß der dritten Ausführungsform wird die Zusammensetzung *in vitro* in einem Gewichtsverhältnis von "1 zu 0,2" bis "1 zu 1" mit Blut vermischt.

In einer neunten Ausführungsform der Zusammensetzung zur Anwendung gemäß einer der Ausführungsformen 3 bis 8 wird ein Gewichtsteil der Zusammensetzung *in vitro* mit 0,5 bis 3,0 Gewichtsteilen Spongiosa oder gereinigten Knochenmarkzellen und mit 0,2 bis 0,8 Gewichtsteilen Blut vermischt.

In einer zehnten Ausführungsform der Zusammensetzung zur Anwendung gemäß einer der Ausführungsformen 3 bis 9 wird das Blut nach dem Vermischen mit der Zusammensetzung einer Gerinnung unterzogen.

In einer elften Ausführungsform der Zusammensetzung zur Anwendung gemäß einer der vorangehenden Ausführungsformen umfasst die Behandlung das Einbringen der Zusammensetzung in einen Knochendefekt, zum Beispiel eine Knochenkavität.

In einer zwölften Ausführungsform der Zusammensetzung zur Anwendung gemäß einer der vorangehenden Ausführungsformen umfasst die Zusammensetzung 70 bis 80 Gewichtsprozent Calciumsulfat.

In einer dreizehnten Ausführungsform der Zusammensetzung zur Anwendung gemäß einer der vorangehenden Ausführungsformen umfasst die Zusammensetzung 12 bis 18 Gewichtsprozent Erdalkalicarbonat.

In einer vierzehnten Ausführungsform der Zusammensetzung zur Anwendung gemäß einer der vorangehenden Ausführungsformen umfasst die Zusammensetzung 7 bis 12 Gewichtsprozent des Bindemittels.

In einer fünfzehnten Ausführungsform der Zusammensetzung zur Anwendung gemäß einer der vorangehenden Ausführungsformen ist der Knochendefekt ein traumatisch, onkologisch oder infektionsbedingt verursachter Knochendefekt.

In einer sechzehnten Ausführungsform liegt die Zusammensetzung als Granulat vor, wobei das Granulat Partikel mit einem mittleren Durchmesser von 1,0 bis 7,0 mm, beispielsweise 0,5 bis 10 mm, 1 bis 3 mm, 2 bis 6 mm, 3 bis 5 mm, oder 3,5 bis 4,5 mm, aufweist.

In einer siebzehnten Ausführungsform weisen die Partikel eine unregelmäßige Form auf, die durch Brechen eines Agglomerats herstellbar ist.

Ein weiterer Aspekt betrifft ein Verfahren zur Herstellung einer Zusammensetzung, umfassend die nachfolgenden Schritte,
- Vermahlen von Calciumsulfat, eines Erdalkalicarbonats, eines Bindemittels, mehr als 0 bis höchstens 2,5 Gewichtsprozent Gentamicinsulfat, bevorzugt 0,5 bis 2,5 Gewichtsprozent Gentamicinsulfat, zu einem Pulver;
- Kompaktieren des Pulvers zu einem Agglomerat;
- Brechen des Agglomerats zu einem Granulat; und
- Fraktionieren des Granulats auf eine gewünschte Partikelgröße.

### AUSFÜHRLICHE BESCHREIBUNG

Zu den hierin beschriebenen Ausführungsformen, deren Elemente ein bestimmtes Merkmal (z.B. ein Material) "aufweisen", oder "umfassen" wird grundsätzlich immer eine weitere Ausführungsform erwogen, in denen das betreffende Element allein aus dem Merkmal besteht, d.h. keine weiteren Bestandteile umfasst. Das Wort "umfassen" oder "umfassend" wird hierin synonym mit dem Wort "aufweisen" oder "aufweisend" verwendet.

Wenn in einer Ausführungsform ein Element mit dem Singular bezeichnet ist, wird ebenfalls eine Ausführungsform erwogen, bei denen mehrere dieser Elemente vorhanden sind. Die Verwendung eines Begriffs für ein Element im Plural umfasst grundsätzlich auch eine Ausführungsform, in welchem nur ein einzelnes entsprechendes Element enthalten ist.

Soweit nicht anders angegeben oder aus dem Zusammenhang eindeutig ausgeschlossen, ist es grundsätzlich möglich und wird hiermit eindeutig in Betracht gezogen, dass Merkmale unterschiedlicher Ausführungsformen auch in den anderen hierin beschriebenen Ausführungsformen vorhanden sein können. Ebenso wird grundsätzlich erwogen, dass alle Merkmale, die hierin in Zusammenhang mit einem Erzeugnis, zum Beispiel einer Zusammensetzung beschrieben werden, auch für die hierin beschriebenen Anwendungen eines solchen Erzeugnisses anwendbar sind, und umgekehrt. Lediglich aus Gründen der knapperen Darstellung werden alle diese erwogenen Kombinationen nicht in allen Fällen explizit aufgeführt. Auch technische Lösungen, die zu den hierin beschriebenen Merkmalen bekanntermaßen gleichwertig sind, sollen grundsätzlich vom Umfang der Erfindung umfasst sein.

Ein Aspekt der Erfindung betrifft eine Zusammensetzung zur Behandlung eines Knochendefekts, welche Calciumsulfat, ein Erdalkalicarbonat, ein Bindemittel, und Gentamicinsulfat umfasst. Bevorzugt umfasst die Zusammensetzung mehr als 0 und höchstens 2,5 Gewichtsprozent Gentamicinsulfat, bezogen auf die Gesamtmasse der Zusammensetzung. In einer Ausführungsform enthält die Zusammensetzung 0,5 bis 2,5 Gewichtsprozent Gentamicinsulfat, bezogen auf die Gesamtmasse der Zusammensetzung. In einer Ausführungsform umfasst die Zusammensetzung 1,2 bis 2,0 Gewichtsprozent Gentamicinsulfat. In einer Ausführungsform umfasst die Zusammensetzung 1,4 bis 1,8 Gewichtsprozent Gentamicinsulfat. In einer Ausführungsform umfasst die Zusammensetzung 1,5 bis 1,7 Gewichtsprozent Gentamicinsulfat, oder 1,55 bis 1,65 Gewichtsprozent, bezogen auf die Gesamtmasse der Zusammensetzung. In einigen Ausführungsformen umfasst die Zusammensetzung eine wirksame Menge Gentamicinsulfat, welche höchstens 2,5 Gewichtsprozent Gentamicinsulfat, bezogen auf die Gesamtmasse der Zusammensetzung, umfasst.

Gentamicin ist ein Aminoglykosidkomplex, der durch Fermentation von *Micromonospora purpurea* oder *Micromonospora echinospora* herstellbar ist. Es wird in seiner Wirkung als Antibiotikum üblicherweise als Sulfatsalz eingesetzt, dem Gentamicinsulfat. Gentamicin bewirkt durch die Bindung an die 30S-Ribosomenuntereinheit das fehlerhafte Ablesen der Codons, indem die Translokation der Peptidyl-tRNA von der Akzeptor- zur Donorseite blockiert wird.

Eine "wirksame Menge" Gentamicinsulfat kann eine Menge sein, die ausreicht, um eine hierin beschriebene Zusammensetzung bereitzustellen, die zumindest die Wachstumsrate von mikrobiellen Organismen im Bereich der Zusammensetzung im Vergleich zu einer Kontrolle reduziert. In vielen Ausführungsformen reicht die Menge des Gentamicinsulfats aus, um eine Hemmzone mit einem Durchmesser von mindestens etwa 10 mm, in der Regel mindestens etwa 15 mm, zu erzeugen, gemessen mit dem Antibiotika-Aktivitätstest gemäß U.S.-Patent Nr. 5,968,253.

In einigen Ausführungsformen enthält eine erfindungsgemäße Zusammensetzung eine Menge an Gentamicinsulfat, welche hoch genug ist, um eine antimikrobielle Wirkung im Patienten zu entfalten, aber ausreichend niedrig ist, um keine cytotoxischen Effekte hervorzurufen.

In einer Ausführungsform umfasst die Zusammensetzung Partikel, welche die oben genannten Bestandteile, nämlich Calciumsulfat, ein Erdalkalicarbonat, ein Bindemittel und Gentamicinsulfat, enthalten. Eine solche Zusammensetzung wird hierin auch als "Granulat", und die einzelnen Partikel werden auch als "Granula" bezeichnet. Bevorzugt liegen das Calciumsulfat, das Erdalkalicarbonat, das Bindemittel und das Gentamicinsulfat gemeinsam in den Partikeln vor. Dies bedeutet, dass die Partikel jeweils ein Gemisch aus den genannten Bestandteilen umfassen. Bevorzugt handelt es sich hierbei um ein homogenes Gemisch, in denen die genannten Bestandteile, insbesondere das Calciumsulfat, das Erdalkalicarbonat, und das Gentamicinsulfat, gleichmäßig verteilt sind.

Die Partikel können eine unregelmäßige Form aufweisen. Hiermit ist gemeint, dass die Partikel insbesondere keine perfekte kugelförmige oder ellipsoide Geometrie aufweisen, sondern an ihrer Oberfläche unregelmäßige Kanten aufweisen. Solche Partikel lassen sich beispielsweise durch das Brechen von Agglomeraten herstellen. Eine solche unregelmäßige Form erleichtert die Verbindung der Partikel miteinander, gegebenenfalls mit weiteren Komponenten, beispielsweise zur Verfüllung einer Knochenkavität.

Zudem kann ein solches Granulat, insbesondere eines mit unregelmäßig geformten Partikeln, vorteilhafte Freisetzungseigenschaften bezüglich des enthaltenen Calciums und/oder des enthaltenen Gentamicins aufweisen. Hierdurch kann eine bessere Wirksamkeit und/oder Verträglichkeit der Zusammensetzung erreicht werden. Diese veränderten Eigenschaften sind auf die im Vergleich zu regelmäßig geformten, beispielsweise zylindrisch oder kugelförmig gepressten, Tabletten auf die veränderten Oberflächeneigenschaften eines solchen Granulats zurückzuführen.

Als "Knochenkavität" wird hierin ein Knochendefekt bezeichnet, der einen Hohlraum in einem Knochen umfasst. Eine solche Knochenkavität kann beispielsweise aufgrund einer Verletzung (im Fachgebiet auch als "Trauma" bezeichnet), durch eine infektionsbedingte Entzündung, oder aufgrund einer anderen Erkrankung des Knochens entstanden sein. Auch ein chirurgischer Eingriff, wie zum Beispiel ein Debridement, können Knochenkavitäten und andere Knochendefekte hervorrufen.

Als "Agglomerate" werden hierin insbesondere Formen verstanden, die durch Verdichten oder Pressen von Pulvern herstellbar sind. Ein Beispiel für ein Agglomerat ist eine Tablette, die durch Pressen eines Pulvers hergestellt ist.

In einigen Ausführungsformen weisen die Partikel einen mittleren Durchmesser von 1,0 bis 7,0 mm auf. In einigen Ausführungsformen beträgt der mittlere Durchmesser der Partikel 0,5 bis 10 mm, 1 bis 3 mm, 2 bis 6 mm, 3 bis 5 mm, oder 3,5 bis 4,5 mm. Dieser mittlere Durchmesser der Partikel kann durch fraktionierte Siebung bestimmt werden, wie es im Fachgebiet üblich und hierin beispielhaft näher beschrieben ist.

In einigen Ausführungsformen sind die Partikel derart aufgebaut, dass die Bestandteile Calciumsulfat, ein Erdalkalicarbonat und Gentamicinsulfat gemeinsam in Teilchen vorliegen, welche innerhalb der Partikel von dem Bindemittel teilweise oder vollständig umgeben sind. Demnach kann die erfindungsgemäße Zusammensetzung Partikel umfassen, welche Teilchen aus Calciumsulfat, einem Erdalkalicarbonat und Gentamicinsulfat umfassen, wobei die Teilchen von einem Bindemittel umgeben sind. Die aus dem Bindemittel gebildete Umhüllungsschicht um die einzelnen Teilchen kann beispielsweise jeweils unter 100 µm dick sein. Ein solcher Aufbau kann eine verzögerte Freisetzung des Gentamicinsulfats begünstigen, wie hierin andernorts ausführlicher beschrieben.

In einigen Ausführungsformen weisen die Partikel eine im wesentlichen kompakte Struktur auf, die weitgehend frei von Hohlräumen ist. Beispielsweise können die Partikel frei von Hohlräumen sein, die einen Durchmesser von mehr als 50 µm aufweisen.

In einigen Ausführungsformen umfasst die Zusammensetzung 60 bis 90 Gewichtsprozent Calciumsulfat. In einigen Ausführungsformen umfasst die Zusammensetzung 70 bis 80 Gewichtsprozent Calciumsulfat, beispielsweise 70 bis 75 Gewichtsprozent, 75 bis 80 Gewichtsprozent, oder 73 bis 77 Gewichtsprozent Calciumsulfat.

In einigen Ausführungsformen umfasst die Zusammensetzung 10 bis 20 Gewichtsprozent Erdalkalicarbonat, beispielsweise 12 bis 18 Gewichtsprozent oder 14 bis 16 Gewichtsprozent Erdalkalicarbonat. Das Erdalkalicarbonat kann beispielsweise Magnesiumcarbonat oder Calciumcarbonat sein. Das Calciumcarbonat kann kristallines Calciumcarbonat sein. Das Calciumcarbonat kann beispielsweise Calcit, Aragonit oder Dolomit sein.

In einigen Ausführungsformen handelt es sich bei dem Calciumsulfat um Calciumsulfat-Dihydrat (CAS-Nr. 10101-41-4), Calciumsulfat-Hemihydrat (CAS-Nr. 10034-76-1) oder kristallwasserfreies Calciumsulfat (CAS-Nr. 7778-18-9). In einigen Ausführungsformen ist das Calciumsulfat Calciumsulfat-Dihydrat. Das Calciumsulfat kann als kristallines oder amorphes Salz vorliegen.

In einer weiteren Ausführungsform umfasst die Zusammensetzung 5 bis 15 Gewichtsprozent des Bindemittels, beispielsweise 7 bis 12 Gewichtsprozent des Bindemittels. Als Bindemittel kann jede geeignete, pharmazeutisch verträgliche Substanz verwendet werden, die in der Lage ist, die in der Zusammensetzung verwendeten Pulverpartikel der Ausgangsstoffe, insbesondere Calciumsulfat und ein Erdalkalicarbonat, so miteinander zu verbinden, dass sich die Zusammensetzung zu einer Tablette oder einem vergleichbaren Formkörper verbinden lässt, beispielsweise in einer gängigen Tablettiermaschine. Geeignete Bindemittel sind beispielsweise Triglyceride, aber auch andere Bindemittel, insbesondere solche, die in der pharmazeutischen Produktion von Tabletten üblich sind, können verwendet werden. Solche Stoffe sind im Fachgebiet als "Bindemitel" bekannt und werden routinemäßig in der pharmazeutischen Produktion verwendet. Das Bindemittel kann ein organischer oder ein anorganischer Stoff sein. Besonders bevorzugt sind organische Substanzen, die eine vergleichsweise weiche und verformbare Konsistenz aufweisen.

Das Bindemittel kann ein hydrophobes Bindemittel sein. "Hydrophob" bedeutet hierin, dass das Bindemittel nicht mit Wasser mischbar ist. In einigen Ausführungsformen hat das Bindemittel einen Verteilungskoeffizienten logP(Oktanol/Wasser) von mindestens 1,0, mindestens 5,0 oder mindestens 10,0 unter Standardbedingungen. Es kann vorteilhaft sein, dass das Bindemittel einen Schmelzpunkt von mindestens 45 °C aufweist. Das Bindemittel kann beispielsweise ein Triglycerid oder Alkanol sein, wobei das Alkanol bevorzugt mindestens 12 Kohlenstoffatome umfasst. In einigen Ausführungsformen umfasst das Alkanol mindestens 13, 14, 15, oder 16 Kohlenstoffatome.

Triglyceride umfassen 3 Carbonsäuren, häufig auch "Fettsäuren" genannt, die gemeinsam mit einem Glycerinmolekül verestert sind. Beispiele für Triglyceride umfassen Glycerintripalmitat (CAS-Nr. 555-44-2), Glycerintristearat (CAS-Nr. 555-43-1) und Glycerintrilaurat (CAS-Nr. 538-24-9). In einigen Ausführungsformen ist das Bindemittel ein gesättigtes Triglycerid, d. h. ein Triglycerid, welches ausschließlich Fettsäuren mit gesättigtem Kohlenwasserstoffanteil enthält. Solche Fettsäuren enthalten keine Doppelbindungen oder Dreifachbindungen im Kohlenwasserstoffanteil. In einigen Ausführungsformen umfasst das Triglycerid 3 identische Fettsäurereste. In einigen Ausführungsformen umfasst das Triglycerid ausschließlich Fettsäurereste mit einer geraden Anzahl an Kohlenstoffatomen. In einigen Ausführungsformen umfasst das Triglycerid ausschließlich lineare, unverzweigte Fettsäurereste. Die erfindungsgemäßen Zusammensetzungen können ein einziges Triglycerid, oder ein Gemisch unterschiedlicher Triglyceride erhalten, beispielsweise ein Gemisch aus Glycerintripalmitat und Glycerintristearat.

In einigen Ausführungsformen ist das Calciumsulfat ausgewählt aus der Gruppe bestehend aus Calciumsulfat-Dihydrat (CAS-Nr. 10101-41-4), Calciumsulfat-Hemihydrat (CAS-Nr. 10034-76-1) und kristallwasserfreies Calciumsulfat (CAS-Nr. 7778-18-9). In einer Ausführungsform ist das Calciumsulfat Calciumsulfat-Dihydrat.

Hierin werden Zusammensetzungen zur Behandlung eines Knochendefekts beschrieben. In einer solchen Behandlung kann die hierin beschriebene Zusammensetzung als Knochenersatzmaterial verwendet werden. Hierzu kann die hierin beschriebene Zusammensetzung in einem Knochendefekt eingebracht werden. Beispielsweise kann ein Knochendefekt mit der hierin beschriebenen Zusammensetzung aufgefüllt werden. Bei dem Knochendefekt kann es sich insbesondere um eine Knochenkavität handeln. Eine Knochenkavität bezeichnet hierin eine nicht natürlich bedingte Vertiefung oder Höhlung, die sich von der Oberfläche eines Knochens in Richtung des Knocheninnenraums erstreckt. Eine Knochenkavität kann traumatisch bedingt sein, beispielsweise durch einen Unfall des Patienten, oder durch einen ärztlichen Eingriff, insbesondere durch eine chirurgische Behandlung, verursacht sein.

In einigen Ausführungsformen umfasst die Behandlung die Auffüllung eines traumatisch, onkologisch oder infektionsbedingt verursachten Knochendefekts mit der Zusammensetzung. Traumatisch verursachte Knochendefekte können insbesondere durch Einwirkung von mechanischen Kräften auf das Skelettsystem entstehen, wobei infolge der Krafteinwirkung das Knochengewebe brechen und bei sehr großer Krafteinwirkung auch in Knochenfragmente zersplittern kann. Beispiele für Knochendefekte umfassen Trümmerfrakturen, welche häufig chirurgisch ausgeräumt werden, so dass Knochenkavitäten an Stelle der Knochensplitter verbleiben. Wenn diese Knochenkavitäten eine kritische Größe (englisch: "critical size defect") überschreiten, ist ein Verschluss der Knochenkavität durch spontane Knochenneubildung nicht mehr ohne Unterstützung durch geeignete Füllmaterialien möglich. "Knochendefekte von kritischer Größe" werden im Fachgebiet und hierin als solche definiert, die im Laufe des Lebens eines Patienten gemäß der ärztlichen Diagnose nicht mehr spontan heilen können. Diese Knochenkavitäten können mit einer hierin beschriebenen Zusammensetzung, ggf. auch mit Gemischen davon mit Blut, gereinigten Knochenmarkzellen (autolog oder allogen)und/oder allogener oder autologer Spongiosa, aufgefüllt werden. Dementsprechend umfasst die Behandlung in einigen Ausführungsformen die Auffüllung eines Knochendefekts, beispielsweise eines traumatisch verursachten Knochendefekts, mit einer hierin beschriebenen Zusammensetzung, wobei der Knochendefekt eine kritische Größe hat.

Gereinigte Knochenmarkzellen werden auch als Knochenmarkkonzentrat oder BMAC bezeichnet. Sie können durch Filtration und Zentrifugation aus primärem Knochenmark gewonnen werden. Ein Verfahren zur Gewinnung von gereinigten Knochenmarkzellen ist beispielsweise in "Chahla, Jorge et al. "Bone Marrow Aspirate Concentrate Harvesting and Processing Technique." Arthroscopy techniques vol. 6,2 e441-e445. 10 Apr. 2017, doi:10.1016/j.eats.2016.10.024" beschrieben. Gereinigte Knochenmarkzellen umfassen demnach eine höhere Konzentration von Stammzellen als primäres Knochenmark. Ein solches Präparat kann insbesondere Wachstumsfaktoren wie Interleukin-1-Rezeptor-Antagonist (IL-1RA) und mesenchymale Stammzellen sowie weitere Vorläuferzellen umfassen.

Onkologisch verursachte Knochendefekte können durch die chirurgische Entfernung von Knochentumoren, beispielweise Osteosarkome und Enchondrome, oder von Knochenmetastasen entstehen. Diese so entstandenen Knochenkavitäten können ebenfalls mit der erfindungsgemäßen Zusammensetzung, oder Gemischen davon, aufgefüllt werden. Die hierin beschriebenen Zusammensetzungen können auch zur Auffüllung chirurgisch sanierter Knochenzysten verwendet werden.

Mikrobiell verursachte Entzündungen des Knochengewebes, welche auch als Osteitis oder Osteomyelitis bezeichnet werden, können hämatogen oder traumatisch entstehen. Solche Entzündungen werden häufig durch ein Debridement behandelt, welches die chirurgische Entfernung von nekrotischem und infiziertem Gewebe umfasst. Bei einem solchen Debridement entstehen regelmäßig Knochendefekte. Auch solche durch ein Debridement erzeugte Knochendefekte können mit den hierin beschriebenen Zusammensetzungen und deren Gemischen behandelt werden. Diese Behandlung umfasst beispielsweise ein Auffüllen der Knochendefekte mit einer hierin beschriebenen Zusammensetzung, oder einem Gemisch aus einer solchen Zusammensetzung mit lebensfähigen Zellen, wie hierin andernorts ausführlicher erläutert. Demnach umfasst die Behandlung in einigen Ausführungsformen die Behandlung eines Knochendefekts, der durch eine Osteitis oder Osteomyelitis, oder durch eine ärztliche Behandlung einer Osteitis oder Osteomyelitis, bedingt ist. In einigen Ausführungsformen umfasst die Behandlung in einigen Ausführungsformen die Behandlung eines Knochendefekts, der durch eine chirurgische Behandlung bedingt ist, welche Knochengewebe entfernt, wie z.B. ein Debridement.

Der hierin verwendete Begriff "traumatisch, onkologisch oder infektionsbedingt verursachte Knochendefekte" umfassen in Übereinstimmung mit den oben dargestellten Erläuterungen auch solche Knochendefekte, die aufgrund der chirurgischen Behandlung eines traumatischen, onkologischen oder infektionsbedingten Zustands entstehen.

Der zu behandelnde Patient kann ein Wirbeltier, beispielsweise ein Säugetier, oder ein Mensch sein.

Die hierin beschriebenen Zusammensetzungen können eine verzögerte Wirkstofffreisetzung bezüglich des enthaltenen Gentamicinsulfats aufweisen. Dies bedeutet, dass das Gentamicinsulfat über einen längeren Zeitraum kontinuierlich an ein wässriges Außenmedium abgegeben wird, beispielsweise über einen Zeitraum von mehreren Tagen bis zu 2 Wochen. Diese Zusammensetzungen können als antimikrobielle Wirkstoffdepots dienen, bei denen die lokale Abgabe eines antimikrobiellen Wirkstoffs wie zum Beispiel Gentamicinsulfat über einen längeren Zeitraum erwünscht ist. Die betreffenden Zusammensetzungen können auch als Vehikel für die lokale Verabreichung von antimikrobiellen Wirkstoffen an Knochengewebe dienen, insbesondere im Bereich von Knochenkavitäten.

Die hierin beschriebenen Zusammensetzungen können zur Behandlung eines Knochendefekts mit lebensfähigen Zellen gemischt werden. Hierzu kann eine solche Zusammensetzung bevorzugt außerhalb des Körpers des Patienten, d. h. *in vitro,* mit den Zellen gemischt werden. In einigen Ausführungsformen wird hierbei ein homogenes Gemisch erhalten, d. h. die Zellen liegen innerhalb des Gemisches im Wesentlichen gleichmäßig verteilt vor. In einigen Ausführungsformen bilden die lebensfähigen Zellen mit der erfindungsgemäßen Zusammensetzung in dem Gemisch makroskopisch gesehen eine gemeinsame Phase. Das so erhaltene Gemisch kann als Knochenersatzmaterial verwendet werden. Beispielsweise kann das Gemisch in einen Knochendefekt, insbesondere eine Knochenkavität, eines Patienten eingebracht werden, wie hierin beschrieben.

Bei den lebensfähigen Zellen handelt es sich bevorzugt um Zellen, welche geeignet sind, die Heilung eines Knochendefekts zu unterstützen. Beispiele für solche Zellen sind Osteoblasten und Osteozyten. Beispielsweise können die hierin beschriebenen Zusammensetzungen mit Blut, gereinigten Knochenmarkzellen oder Spongiosa gemischt werden. Bei der Verwendung von Blut kann bevorzugt gerinnungsfähiges Blut verwendet werden, zum Beispiel Vollblut. Die lebensfähigen Zellen können von demselben Patienten stammen, der mit dem Gemisch zu behandeln ist. Hierbei spricht man auch von "autologen" Zellen. In einigen Ausführungsformen können auch Zellen von anderen Spendern verwendet werden. Hierbei spricht man auch von "allogenen" Zellen. Dementsprechend können die hierin beschriebenen Zusammensetzungen mit autologen oder allogenem Blut, gereinigten Knochenmarkzellen und/oder mit autologer oder allogener Spongiosa gemischt werden.

Wenn der zu behandelnde Patient ein Mensch ist, kann es sich bei den Zellen bevorzugt um humane Zellen handeln. In entsprechender Weise können die Zellen, die im Zusammenhang mit den hierin beschriebenen Zusammensetzungen zur Anwendung in einem Verfahren zur Behandlung eines Tierpatienten vorgesehen sind, von derselben Spezies wie der Tierpatient stammen.

Die Spongiosa kann in Form von Knochenchips mit der hierin beschriebenen Zusammensetzung gemischt werden. Bevorzugt können Knochenchips verwendet werden, welche eine Länge von 1 bis 10 mm aufweisen, beispielsweise eine Länge von 2 bis 9 mm, 3 bis 8 mm, 4 bis 7 mm, 1 bis 5 mm, oder 2 bis 4 mm. Zur Ermittlung dieser Länge ist jeweils der maximale Durchmesser eines Knochenchips maßgeblich, wobei im Wesentlichen alle Knochenchips einen Durchmesser im Bereich von beispielsweise 1 bis 10 mm aufweisen.

Die hierin verwendete Zusammensetzung kann beispielsweise in einem Gewichtsverhältnis von "1 zu 3" bis "1 zu 0,5" mit Spongiosa oder gereinigten Knochenmarkzellen gemischt werden, d. h. es wird jeweils ein Teil der hierin beschriebenen Zusammensetzung mit 0,5 bis 3 Teilen Spongiosa oder gereinigten Knochenmarkzellen gemischt.

In einigen Ausführungsformen wird eine hierin beschriebene Zusammensetzung in einem Gewichtsverhältnis von "1 zu 0,2" bis "1 zu 1" mit Blut vermischt, d. h. es wird jeweils ein Teil der hierin beschriebenen Zusammensetzung mit 0,2 bis 1 Teilen Blut gemischt. Bei dem Blut kann es sich beispielsweise um Vollblut handeln. Bevorzugt ist das Blut gerinnungsfähig. Ein Gemisch, welches eine hierin beschriebene Zusammensetzung und gerinnungsfähiges Blut enthält, kann bevorzugt nach der Gerinnung des Blutes zur therapeutischen Anwendung verwendet werden, insbesondere zum Auffüllen eines Knochendefekts, wie hierin beschrieben.

In einigen Ausführungsformen wird eine hierin beschriebene Zusammensetzung sowohl mit Spongiosa oder gereinigten Knochenmarkzellen als auch mit Blut vermischt. Hierbei kann ein Gewichtsteil der erfindungsgemäßen Zusammensetzung bevorzugt jeweils mit 0,5 bis 3,0 Gewichtsteilen Spongiosa oder gereinigten Knochenmarkzellen und mit 0,2 bis 0,8 Gewichtsteilen Blut vermischt werden. Das hierdurch gebildete Gemisch kann danach einer Gerinnung des darin enthaltenen Bluts unterzogen werden. Nach erfolgter Blutgerinnung kann das Gemisch an den Patienten verabreicht werden.

Die lebensfähigen Zellen, beispielsweise in Form von Blut, gereinigten Knochenmarkzellen und/oder Spongiosa, können beispielsweise von Hand, unter Verwendung eines Rotationsmischer oder unter Verwendung eines pneumatischen Mischers in die erfindungsgemäßen Zusammensetzungen eingearbeitet werden.

In einigen Ausführungsformen, in welchen die hierin beschriebene Zusammensetzung mit Blut vermischt wird, benetzt das Blut nach dem Vermischen mit der Zusammensetzung die Oberfläche der Zusammensetzung. Hierdurch kann die Verbindung mit bzw. das Anwachsen von patienteneigenen Zellen verbessert werden.

In einigen Ausführungsformen werden die Zellen kurz nach ihrer Entnahme aus dem Spender mit einer hierin beschriebenen Zusammensetzung gemischt, und innerhalb kurzer Zeit dem Patienten verabreicht. Beispielsweise kann diese Verabreichung innerhalb 1 Stunde, oder innerhalb von 10, 20 oder innerhalb von 30 Minuten nach der Entnahme der Zellen aus dem Spender erfolgen. In einer Ausführungsform erfolgen die Entnahme der Zellen aus dem Spender und die Verabreichung an den Patienten innerhalb desselben Operationsvorgangs. Hierbei werden durch den behandelnden Arzt zunächst dem Patienten eigene Zellen entnommen, zum Beispiel in Form von Spongiosa, gereinigten Knochenmarkzellen und/oder Blut, mit einer erfindungsgemäßen Zusammensetzung gemischt, und anschließend mit möglichst kurzem Zeitabstand, gegebenenfalls nach Gerinnung des Blutes, dem Patienten im Bereich eines Knochendefekts verabreicht.

Die erfindungsgemäßen Zusammensetzungen, sowie ihre Gemische mit lebensfähigen Zellen, können von Hand oder mithilfe einer geeigneten Austragsvorrichtung, wie zum Beispiel einer manuell bedienbaren Kolbenauspressvorrichtung oder Spritzen-artigen Vorrichtung, an einen Patienten verabreicht werden, insbesondere im Bereich eines Knochendefekts wie zum Beispiel eine Knochenkavität.

Bei der hierin beschriebenen Zusammensetzung zur Anwendung bei der Behandlung eines Knochendefekts ist es vorteilhaft, wenn der Gehalt an Gentamicinsulfat nicht zu cytotoxischen Effekten führt. Beispielsweise sollen im Bereich der Verabreichung der Zusammensetzung, beispielsweise innerhalb eines Knochendefekts, bevorzugt keine cytotoxischen Effekte auftreten. Solche cytotoxischen Effekte umfassen beispielsweise die Lyse von Blutzellen, wie zum Beispiel Leukozyten oder Erythrozyten, beim Kontakt mit einem Knochenersatzmaterial. Dies ist insbesondere dann relevant, wenn die Zusammensetzung *in vitro* mit lebensfähigen Zellen gemischt wird, wie vorangehend beschrieben. Eine erfindungsgemäßer Gehalt an Gentamicinsulfat kann bewirken, dass die lebensfähigen Zellen ihre Fähigkeit zur Regeneration von Knochengewebe nach dem Einbringen in eine Knochenkavität eines zu behandelnden Patienten weiterhin aufrechterhalten, was zu einem verbesserten Therapieerfolg führen kann. Hierzu kann ein Gentamicinsulfat-Gehalt von nicht mehr als 2,5 Gewichtsprozent, bezogen auf das Gesamtgewicht der erfindungsgemäßen Zusammensetzung, vorteilhaft sein. In einigen Ausführungsformen sind die hierin beschriebenen Zusammensetzungen nicht cytotoxisch gemäß der Norm ISO10993-5. In einigen Ausführungsformen sind die hierin beschriebenen Zusammensetzungen und deren hierin beschriebene Gemische nicht cytotoxisch gemäß dem In-vitro-Cytotoxizitätstest der Norm ISO10993-5. In einigen Ausführungsformen sind die hierin beschriebenen Zusammensetzungen und deren hierin beschriebene Gemische nicht cytotoxisch gemäß dem Test für akute systemische Toxizität gemäß der Norm ISO10993-5.

Auch mikrobielle Besiedelung des Knochenersatzmaterials kann die Fähigkeit zur Regeneration von Knochengewebe beeinträchtigen. Eine wirksame Konzentration von Gentamicinsulfat kann daher durch seine antimikrobielle Wirkung zu einem verbesserten Therapieerfolg das erfindungsgemäßen Knochenersatzmaterials beitragen. Die antimikrobielle Wirkung des Gentamicinsulfats kann sowohl das Knochenersatzmaterial, als auch dessen Einsatzort im Patienten vor Besiedlung mit mikrobiellen Keimen schützen.

Auch die hierin beschriebene verzögerte Freisetzung kann zu einem verbesserten Therapieerfolg beitragen, indem einer mikrobiellen Besiedlung und daraus potenziell resultierenden Infektionen über längere Zeit vorgebeugt werden kann.

In einigen Ausführungsformen weisen die hierin beschriebenen Zusammensetzungen oder Gemische davon die Fähigkeit auf, als geeignetes Trägermaterial zu dienen, an der Knochengewebe wachsen kann.

In einigen Ausführungsformen weisen die hierin beschriebenen Zusammensetzungen oder Gemische davon eine osteogene Wirkung auf. Der Begriff "osteogen" bezieht sich hier auf die Fähigkeit eines Materials, das Wachstum von neuem Knochengewebe durch einen oder mehrere Mechanismen wie zum Beispiel Osteogenese, Osteokonduktion und/oder Osteoinduktion zu fördern und/oder zu beschleunigen.

Zusätzlich zu den oben genannten Bestandteilen können die erfindungsgemäßen Zusammensetzungen außerdem eine weitere therapeutische Substanz umfassen, z.B. Wirkstoffe zur Induktion des Knochenwachstums wie knochenmorphogenetische Proteine, Wachstumsfaktoren, Peptide und dergleichen, antivirale Mittel, Antibiotika usw.); oder monofile oder multifile Strukturen, Folien, Beschichtungen, Membranen (z. B. porös, mikroporös, resorbierbar usw.), Schaumstoffe (z. B. offenzellig oder geschlossenzellig), Schraubenaugmentationen, Schädelrekonstruktionen und/oder Kombinationen davon umfassen. Die therapeutische Substanz kann in den erfindungsgemäßen Zusammensetzungen enthalten sein. Die therapeutischen Substanzen können andere Antibiotika, Chemotherapeutika, Wachstumsfaktoren (insbesondere osteoinduktive Wachstumsfaktoren) wie knochenmorphogenetische Proteine, endotheliale Wachstumsfaktoren, Insulin-Wachstumsfaktoren oder Ähnliches oder eine Kombination davon umfassen, sind aber nicht darauf beschränkt. Nicht einschränkende Beispiele für verwendbare weitere antimikrobielle Wirkstoffe sind: Antiamebika, z. B. Arsthinol, Bialamicol, Carbarsone, Cephaeline, Chlorbetamide, Chloroquin, Chlorphenoxamide, Chlortetracyclin, Dehydroemetine, Dibromopropamidine, Diloxanide, Diphetarsone, Emetine, Fumagillin, Glaucarubin, Glycobiarsol, 8-Hydroxy-7-iodo-5-chinolin-sulfonsäure, lodochlorhydroxychin, lodochinol, Paromomycin, Phanchinon, Polybenzarsol, Propamidin, Quinfamid, Scenidazol, Sulfarsid, Teclozan, Tetracyclin, Thiocarbamizin, Thiocarbarsone, Tinidazol; Antibiotika, z.B. Aminoglykoside (wie Amikacin, Apramycin, Arbekacin, Bambermycine, Butirosin, Dibekacin, Dihydrostreptomycin, Fortimicin (s),Isepamicin, Kaniamycin, Micronomicin, Neomycin, Neomycin Undecylenat, Netilmicin, Paromomycin, Ribostamycin, Sisomicin, Spectinomycin, Streptomycin, Tobramycin, Trospectomycin), Amphenicole (Azidamfenicol, Chloramphenicol, Florfenicol, Thiamphenicol), Ansamycine (Rifamid, Rifampin, Rifamycin, Rifapentin, Rifaximin), Lactame (Carbacepheme, Loracarbef, Carbapeneme (Biapenem, Imipenem, Meropenem, Panipenem), Cephalosporine (Cefaclor, Cefadroxil, Cefamandol, Cefatrizin, Cefazedon, Cefazolin, Cefcapen Povoxil, Cefclidin, Cefdinir, Cefditoren, Cefepime, Cefetamet, Cefixime, Cefinenoxin, Cefodizim, Cefonicid, Cefoperazon, Ceforanid, Cefotaxim, Cefotiam, Cefozopran, Cefpimizol, Cefpiramid, Cefpirom, Cefpodoxim Proxetil, Cefprozil, Cefroxadin, Cefsulodin, Ceftazidim, Cefteram, Ceftezol, Ceftibuten, Ceftizoxim, Ceftriaxon, Cefuroxim, Cefuzonam, Cephacetril-Natrium, Cephalexin, Cephaloglycin, Cephaloridin, Cephalosporin, Cephalothin, Cephapirin-Natrium, Cephradin, Pivcefalexin), Cephamycine (Cefbuperazon, Cefmetazol, Cefminox, Cefotetan, Cefoxitin), Monobactame (Aztreonam, Carumonam, Tigemonam), Oxacephene (Flomoxef, Moxalactam), Penicilline (Amdinocillin, Amdinocillin Pivoxil, Amoxicillin, Ampicillin, Apalcillin, Aspoxicillin, Azidocillin, Azlocillin, Bacampicillin, Benzylpenicillinsäure, Benzylpenicillin-Natrium, Carbenicillin, Carindacillin, Clometocillin, Cloxacillin, Cyclacillin, Dicloxacillin, Epicillin, Fenbenicillin, Floxacillin, Hetacillin, Lenampicillin, Metampicillin, Methicillin-Natrium, Mezlocillin, Nafcillin-Natrium, Oxacillin, Penamecillin, Penethamat-Hydriodid, Penicillin G Benethamin, Penicillin G Benzathin, Penicillin G Benzhydrylamine, Penicillin G Calcium, Penicillin G Hydrabamine, Penicillin G Kalium, Penicillin G Procaine, Penicillin N, Penicillin O, Penicillin V, Penicillin V Benzathine, Penicillin V Hydrabamin, Penimepicyclin, Phenethicillin Kalium, Piperacillin, Pivampicillin, Propicillin, Quinacillin, Sulbenicillin, Sultamicillin, Talampicillin, Temocillin, Ticarcillin), Ritipenem), Lincosamide (Clindamycin, Lincomycin), Makrolide (Azithromycin, Carbomycin, Clarithromycin, Dirithromycin, Erythromycin, Erythromycin Acistrate, Erythromycin Estolat, Erythromycin Glucoheptonat, Erythromycin Lactobionat, Erythromycin Propionat, Erythromycin Stearat, Josamycin, Leucomycine, Midecamycine, Miokamycin, Oleandomycin, Primycin, Rokitamycin, Rosaramicin, Roxithromycin, Spiramycin, Troleandomycin), Polypeptide (Amphomycin, Bacitracin, Capreomycin, Colistin, Enduracidin, Enviomycin, Fusafungin, Gramicidin S, Gramicidin (s), Mikamycin, Polymyxin, Pristinamycin, Ristocetin, Teicoplanin, Thiostrepton, Tuberactinomycin, Tyrocidin, Tyrothricin, Vancomycin, Viomycin, Virginiamycin, Zink-Bacitracin), Tetracycline (Apicyclin, Chlortetracyclin, Clomocyclin, Demeclocyclin, Doxycyclin, Guamecyclin, Lymecyclin, Meclocyclin, Methacyclin, Minocyclin, Oxytetracyclin, Penimepicyclin, Pipacyclin, Rolitetracyclin, Sancyclin, Tetracyclin), Cycloserin, Mupirocin, Tuberin; synthetische antibakterielle Mittel, z. B. 2,4-Diaminopyrimidine (Brodimoprim, Textroxoprim, Trimethoprim), Nitrofurane (Furaltadon, Furazoliumchlorid, Nifuradene, Nifuratel, Nifurfoline, Nifurpirinol, Nifurprazin, Nifurtoinol, Nitrofirantoin), Chinolone und Analoga (Cinoxacin, Ciprofloxacin, Clinafloxacin, Difloxacin, Enoxacin, Fleroxacin, Flumequin, Grepafloxacin, Lomefloxacin, Miloxacin, Nadifloxacin, Nadilixinsäure, Norflaxacin, Ofloxacin, Oxolinsäure, Pazufloxacin, Pefloxacin, Pipemidinsäure, Piromidinsäure, Rosoxacin, Rufloxacin, Sparfloxacin, Temafloxacin, Tosufloxacin, Trovafloxacin), Sulfonamide (Acetylsulfamethoxpyrazin, Benzylsulfamid, Chloramin- B, Chloramin-T, Dichloramin T, N2-Formylsulfisomidin, N4-beta-D-Glucosylsulfanilamid, Mafenid, 4'-(Methylsulfamoyl)sulfanilanilid, Noprylsulfamid, Phthalylsulfacetamid, Phthalylsulfathiazol, Salazosulfadimidin, Succinylsulfathiazol, Sulfabenzamid, Sulfacetamid, Sulfachlorpyridazin, Sulfachrysoidin, Sulfacytin, Sulfadiazin, Sulfadicramid, Sulfadimethoxin, Sulfadoxin, Sulfaethidol, Sulfaguanidin, Sulfaguanol, Sulfalene, Sulfaloxic, Sulfamerazin, Sulfameter, Sulfamethazin, Sulfamethizol, Sulfamethomidin, Sulfamethoxazol, Sulfamethoxypyridazin, Sulfametrole, Sulfamidochrysoidin, Sulfamoxol, Sulfanilamid, 4-Sulfanilamidosalicylsäure, N4-Sulfanilylsulfanilamid, Sulfanilylharnstoff, N-Sulfanilyl- 3,4-xylamid, Sulfanitran, Sulfaperin, Sulfaphenazol, Sulfaproxylin, Sulfapyrazin, Sulfapyridin, Sulfasomizol, Sulfasymazin, Sulfathiazol, Sulfathioharnstoff, Sulfatolamid, Sulfisomidin, Sulfisoxazol), Sulfone (Acedapson, Acediasulfon, Acetosulfon-Natrium, Dapson, Diathymosulfon, Glucosulfon-Natrium, Solasulfon, Succisulfon, Sulfanilsäure, p-Sulfanilylbenzylamin, Sulfoxon-Natrium, Thiazolsulfon), Clofoctol, Hexedin, Methenamin, Methenamin-Anhydromethylencitrat, Methenamin-Hippurat, Methenamin-Mandelat, Methenamin-Sulfosalicylat, Nitroxolin, Taurolidin, Xibomol; leprostatische antibakterielle Mittel, wie Acedapson, Acetosulfon-Natrium, Clofazimin, Dapson, Diathymosulfon, Glucosulfon-Natrium, Hydnocarpinsäure, Solasulfon, Succisulfon, Sulfoxon-Natrium, Antimykotika, wie Allylamine, Butenafin, Naftifin, Terbinafin, Imidazole (z. g., Bifonazol, Butoconazol, Cholordantoin, Chlormidazol, Cloconazol, Clotrimazol, Econazol, Enilconazol, Fenticonazol, Flutrimazol, Isoconazol, Ketoconazol, Lanoconazol, Miconazol, Omoconazol, Oxiconazolnitrat, Sertaconazol, Sulconazol, Tioconazol), Thiocarbamate (Tolcilat, Tolindat, Tolnaftat), Triazole (Fluconazol, Itraconazol, Saperconazol, Terconazol), Acrisorcin, Amorolfin, Biphenamin, Bromosalicylchloranilid, Buclosamid, Calciumpropionat, Chlorphenesin, Ciclopirox, Cloxyquin, Coparaffinat, Diamthazoldihydrochlorid, Exalamid, Flucytosin, Halethazol, Hexetidin, Loflucarban, Nifuratel, Kaliumjodid, Propionsäure, Pyrithion, Salicylanilid, Natriumpropionat, Sulbentin, Tenonitrozol, Triacetin, Ujothion, Undecylensäure, Zinkpropionat; und dergleichen.

Andere weitere antimikrobielle Mittel, die für die vorliegende Erfindung verwendbar sind, umfassen p-Lactamase-Inhibitoren (z. B. Clavulansäure, Sulbactam, Tazobactam); Chloramphenicole (z. B. Azidamphenicol, Chloramphenicol, Thiaphenicol); Fusidinsäure; synthetische Mittel wie Trimethoprim, gegebenenfalls in Kombination mit Sulfonamiden) und Nitroimidazole (z. B., Metronidazol, Tinidazol, Nimorazol); antimykobakterielle Mittel (z. B. Capreomycin, Clofazimin, Dapson, Ethambutol, Isoniazid, Pyrazinamid, Rifabutin, Rifampicin, Streptomycin, Thioamide); antivirale Mittel (z. B. Acryclovir, Amantadin, Azidothymidin, Ganciclovir, Idoxuridin, Tribavirin, Trifluridin, Vidarabin); Interferone (z. B. Interferon a, Interferon p); und antiseptische Mittel (z. B. Chlorhexidin, Enzianviolett, Octenidin, Povidon-Jod, quaternäre Ammoniumverbindungen, Silbersulfadiazin, Triclosan).

Das weitere antimikrobielle Mittel kann Mittel umfassen, die Krankheiten behandeln, die durch grampositive und/oder gramnegative Bakterien verursacht werden. Bevorzugte antimikrobielle Mittel sind unter anderem Amikacin, Tobramycin, Vancomycin und deren Salze.

Die therapeutische Substanz kann ferner eine biologische therapeutische Substanz, wie z. B. ein Protein, enthalten. Knochenassoziierte Proteine können zugesetzt werden, um die physikalischen Eigenschaften der Zusammensetzung zu verändern, Resorption, Angiogenese, Zelleintritt und -proliferation, Mineralisierung, Knochenbildung, Wachstum von Osteoklasten und/oder Osteoblasten zu fördern, oder ähnliches. Proteine von besonderem Interesse sind die verschiedenen Arten von Kollagen, insbesondere Typ I. Andere verwendbare Proteine sind Osteonectin, Knochensialoproteine (Bsp), alpha-2HS-Glykoproteine, Knochen-Gla-Protein (Bgp), Matrix-Gla-Protein, Knochen-Phosphoglykoprotein, Knochen-Phosphoprotein, Knochen-Proteoglykan, Protolipide, morphogene Knochenproteine (z. B., BMP-1, - 2A, -2B, -3,-3b,-4,-5,-6,-7,-8,-8b,-9,-10,-11,-12,-13,-14,-15), Knorpelinduktionsfaktor, Platelet Derived Growth Factor (PDGF-, -2), Endothelial Cell Growth Factor (ECGF- 1, -2a, -2b), Skeletal Growth Factor (IGF-2), insulinähnliche Wachstumsfaktoren (IGF-1, IGF- 2), Fibroblasten-Wachstumsfaktor (ODGF-1,-2,-3,-4,-5,-6,-7,-8,-9,-10,-11,-12,-13,-14,-15, -16,-17,-18,-19,-20,-21,-22,-23), Koloniestimulierender Faktor, Transformierender Wachstumsfaktor (z.B. TGF-, B), vaskuläre endotheliale Wachstumsfaktoren (VEGF), Wachstums-/Differenzierungsfaktoren (GDF-1, -3, -5,-6,-7,-8,-9,-9B,-10,-1 1,-15,-16), osteogene Proteine (BMP-7, BMP-8, BMP-8b), braunes Wachstumshormon, Parathormon (PTH), Insulin, Calcitonin und dergleichen. Zu den Proteinen können auch mit Knorpel assoziierte Proteine wie das Chondrokalzinierungsprotein, mit Dentin assoziierte Proteine wie Phosphophoryn, Glykoproteine und Gla-Proteine oder mit Schmelz assoziierte Proteine wie Amelognin und Enamelin gehören. Zu den interessanten Strukturproteinen gehören Fibrin, Fibrinogen, Keratin, Tubulin, Elastin und dergleichen. Blutproteine können einzeln oder zusammen in Plasma oder Serum verwendet werden, z. B. Serumalbumin.

Die therapeutische Substanz kann ferner einen Nicht-Protein-Wachstumsfaktor wie Prostaglandine und Statine (z. B. Simvastatin, Lovastatin) umfassen.

In einer Ausführungsform handelt es sich bei der therapeutischen Substanz um einen Wachstumsfaktor, wie z. B. knochenmorphogenetische Proteine, Wachstumsfaktoren für endotheliale Zellen, insulinähnliche Wachstumsfaktoren oder dergleichen oder eine Kombination davon.

Bevorzugt wird die weitere therapeutische Substanz gegebenenfalls in einer nicht cytotoxischen Menge in der hierin beschriebenen Zusammensetzung verwendet.

Ein weiterer Aspekt betrifft ein Verfahren zur Herstellung einer Zusammensetzung, umfassend die nachfolgenden Schritte,
- Vermahlen von Calciumsulfat, eines Erdalkalicarbonats, eines Bindemittels, und mehr als 0 bis 2,5 Gewichtsprozent Gentamicinsulfat, beispielsweise 0,5 bis 2,5 Gewichtsprozent Gentamicinsulfat, bezogen auf die Gesamtmasse der Zusammensetzung, zu einem Pulver;
- Kompaktieren des Pulvers zu einem Agglomerat;
- Brechen des Agglomerats zu einem Granulat; und
- Fraktionieren des Granulats auf eine gewünschte Partikelgröße.

Die Ausgangsstoffe können grundsätzlich in einer beliebigen festen Form vorliegen, die einer Vermahlung unterzogen werden kann. Beim Vermahlen können bevorzugt Mahlkörper verwendet werden, zum Beispiel Mahlkugeln, welche beispielsweise aus keramischen Materialien, wie Porzellan oder Korund, bestehen können. Durch das Vermahlen wird ein Pulver der oben genannten Ausgangsstoffe erhalten. Dieses Pulver stellt bevorzugt ein homogenisiertes Gemisch aus diesen Ausgangsstoffen dar.

Anschließend kann das so erhaltene Pulver kompaktiert werden. Das Kompaktieren kann durch jedes im Fachgebiet übliche und geeignete Verfahren erfolgen, zum Beispiel mit üblichen Rundläufertablettierpressen oder Exzenterpressen. Das Kompaktieren kann beispielsweise durch Verpressen des Pulvers mit einer Kraft von mindestens 30 kN erfolgen. Durch das Kompaktieren wird aus dem Pulver ein kompakter Formkörper, wie zum Beispiel eine Tablette, erhalten. Ein solcher Formkörper kann eine regelmäßige Gestalt aufweisen, beispielsweise die Form eines Zylinders, einer Kugel oder eines Ovoids. Ein solcher Formkörper wird hierin auch als "Agglomerat" bezeichnet. Ein Beispiel für einen Agglomerat ist eine Tablette, die durch Verpressen eines Pulvers herstellbar ist.

Durch das Brechen des Agglomerates kann ein Granulat erhalten werden. Das Brechen des Agglomerates kann beispielsweise mithilfe eines Brechsiebs oder einer Siebmühle erfolgen, wie im Fachgebiet bekannt und üblich. Die einzelnen Granula (d. h. Körner) eines solchen Granulats weisen häufig eine zufällige Gestalt und Größe auf. Das Granulat kann beispielsweise mithilfe eines Siebturms fraktioniert werden, wie es im Fachgebiet üblich und in den nachfolgenden Beispielen beschrieben ist. Hierdurch kann ein Granulat mit einer gewünschten Partikelgrößenfraktion erhalten werden. Die gewünschte Partikelgröße beträgt beispielsweise 1 bis 7 mm, wie hierin andernorts ausführlicher beschrieben.

Bevorzugt wird das Verfahren unter Bedingungen durchgeführt, welche ein Schmelzen des Bindemittels vermeiden. Hierzu kann beispielsweise bei Umgebungsbedingungen unterhalb der Schmelztemperatur des Bindemittels gearbeitet werden, und/oder bei den genannten Prozessschritten, insbesondere dem Vermahlen, die Wärmeentwicklung entsprechend überwacht und gesteuert werden.

Ein weiterer Aspekt betrifft ein Granulat, welches gemäß dem oben beschriebenen Verfahren herstellbar ist. Das Granulat kann Calciumsulfat, ein Erdalkalicarbonat, ein Bindemittel, und 0,5 bis 2,5 Gewichtsprozent Gentamicinsulfat, bezogen auf die Gesamtmasse der Zusammensetzung, umfassen.

Ein weiterer Aspekt betrifft ein Agglomerat, umfassend Calciumsulfat, ein Erdalkalicarbonat, ein Bindemittel, und mehr als 0 bis 2,5 Gewichtsprozent Gentamicinsulfat, bezogen auf die Gesamtmasse der Zusammensetzung. Das Agglomerat kann bevorzugt 0,5 bis 2,5 Gewichtsprozent Gentamicinsulfat umfassen, beispielsweise 1,2 bis 2,0; 1,4 bis 1,8 %; oder 1,5 bis 1,7 %, oder 1,55 % bis 1,65 %.

Die Agglomerate sind, wie hierin zuvor ausführlicher beschrieben, durch Verdichten eines Pulvers herstellbar. Die Agglomerate können beispielsweise eine zylindrische Form mit einer Höhe von 5 bis 15 mm und einem Durchmesser von 15 bis 40 mm aufweisen. Die Agglomerate können auch eine kugelförmige oder ovoide Form mit vergleichbarer Größe aufweisen.

Die Agglomerate können gegebenenfalls zu irregulär geformten Granulaten gebrochen werden, um ein hierin beschriebenes Knochenersatzmaterial zu erhalten.

In einigen Ausführungsformen enthalten die hierin beschriebenen Zusammensetzungen oder Agglomerate beispielsweise 1,6 Gew-% Gentamicinsulfat. Das bedeutet, dass 1,0 Gew.-% Gentamicinbase bei einem Aktivitätskoeffizienten von AK= 600 des Gentamicinsulfats in der Zusammensetzung oder dem verdichteten Gemisch enthalten sind. Ein Aktivitätskoeffizient von 600 bedeutet, dass in 1 mg Gentamicinsulfat 600 µg Gentamicinbase vorliegen.

Ein weiterer Aspekt betrifft ein oben beschriebenes Granulat oder Agglomerat zur Anwendung in einem Verfahren zur Behandlung eines Knochendefekts.

Ein weiterer Aspekt der Erfindung betrifft eine Zusammensetzung, umfassend Calciumsulfat, ein Erdalkalicarbonat, ein Bindemittel, mehr als 0 bis höchstens 2,5 Gewichtsprozent Gentamicinsulfat, bezogen auf die Gesamtmasse der Zusammensetzung.

Eine zweite Ausführungsform dieses Aspekts betrifft eine Zusammensetzung, umfassend Partikel, in welchen das Calciumsulfat, das Bindemittel, das Erdalkalicarbonat und das Gentamicinsulfat gemeinsam vorliegen.

Hierbei können die Partikel eine unregelmäßige Form aufweisen, welche durch Brechen von Agglomeraten herstellbar ist.

Die Partikel können einen mittleren Durchmesser von 1,0 bis 7,0 mm aufweisen, wobei der mittlere Durchmesser der Partikel durch fraktionierte Siebung bestimmbar ist.

Die Zusammensetzung kann beispielsweise 70 bis 80 Gewichtsprozent Calciumsulfat umfassen.

Die Zusammensetzung kann 10 bis 20, oder bevorzugt 12 bis 18 Gewichtsprozent Erdalkalicarbonat umfassen.

Die Zusammensetzung kann 5 bis 15, bevorzugt 7 bis 12 Gewichtsprozent des Bindemittels umfassen.

Das Bindemittel kann einen Schmelzpunkt von mindestens 45 °C aufweisen.

Das Bindemittel kann ein Triglycerid oder Alkanol sein, wobei das Alkanol bevorzugt mindestens 12 Kohlenstoffatome umfasst.

Das Calciumsulfat kann ausgewählt sein aus der Gruppe bestehend aus Calciumsulfat-Dihydrat, Calciumsulfat-Hemihydrat, und kristallwasserfreiem Calciumsulfat.

Das Erdalkalicarbonat kann ausgewählt sein aus der Gruppe bestehend aus Calcit, Aragonit, Magnesiumcarbonat und Dolomit.

Ein weiterer Aspekt der Erfindung betrifft ein Therapieverfahren, in welchem eine hierin beschriebene Zusammensetzung wie hierin beschrieben an einen Patienten verabreicht wird. Das Therapieverfahren ist bevorzugt die Behandlung eines Knochendefekts.

### BEISPIELE

Die Erfindung wird nachfolgend anhand von Beispielen weiter verdeutlicht, die jedoch nicht als einschränkend zu verstehen sind. Dem Fachmann wird ersichtlich sein, dass anstelle der hier beschriebenen Merkmale andere äquivalente Mittel in ähnlicher Weise verwendet werden können.

Für die nachfolgenden Beispiele wurde Calciumsulfat-Dihydrat (CS), Calciumcarbonat (CC), Magnesiumcarbonat (MC), Glycerintriplamitat (GTP), Glycerintristearat (GTS) Gentamicinsulfat ((GS), Aktivitätskoeffizient AK= 600) der Firma Sigma-Aldrich verwendet. Das Glycerintriplamitat (GTP) wurde als feines Pulver mit einer Partikelgröße deutlich kleiner 1 mm eingesetzt.

### BEISPIELE 1 BIS 7: HERSTELLUNG VON KNOCHENERSATZMATERIALIEN

Die Gemische der in der Tabelle dargestellten Beispiele 1-7 wurden mit einem Turbula-Mischer unter Verwendung von Porzellanmahlkugeln innerhalb von 10 Stunden homogenisiert. Anschließend wurden die homogenisierten Gemische mit einer Exzenter-Tablettierpresse zu zylindrisch Formkörpern mit einer Höhe von h= 10 mm, einem Durchmesser von 25 mm gepresst. Die zylindrischen Formkörper wurden mit einem oszillierenden Frewitt-Brechsieb zerkleinert und das erhaltene Granulat mit einem Siebturm in die Fraktionen 1-3 mm und 3-5 mm fraktioniert. Das Gewichtsverhältnis von Calciumsulfat-Dihydrat zu Calciumcarbonat/Magnesiumcarbonat zu Glycerintripalmitat/Glycerintristearat wurde konstant gelassen und der Gehalt an Gentamicinsulfat zwischen 0,5 Gew.-% und 2,5 Gew.-% variiert.

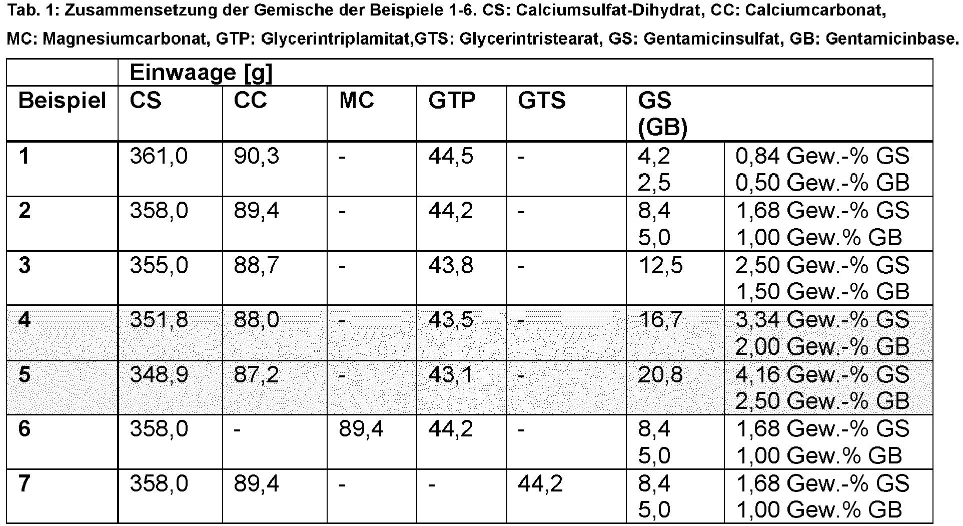

Die *in-vitro*-Cytotoxizität der Probekörper der Beispiele 1-6 wurde gemäß der ISO1099-5 geprüft. Nicht cytotoxisch waren die Probekörper der Beispiele 1-3, 6 und 7. Die Probekörper der Beispiele 4 und 5 zeigten einen cytotoxischen Effekt.

In den folgenden Beispielen wurde die Vermischbarkeit des erfindungsgemäßen Knochenersatzmaterials mit Vollblut und mit allogenen Knochenchips (allogener Spongiosa) geprüft.

### BEISPIEL 8

Es wurde 5,0 g Granulat der Fraktion 1-3 mm des Beispiels 2 mit 1,0 ml (~1,0 g) frisches Volblut (Schweineblut) in einer Nierenschale miteinander vermischt. Nach rund 10 Minuten wurde nach Gerinnung des Vollbluts eine gelartige Masse erhalten.

### BEISPIEL 9

Es wurde 5,0 g Granulat der Fraktion 1-3 mm des Beispiels 2 mit 5,0 ml (~5,0 g) frisches Vollblut (Schweineblut) in einer Nierenschale miteinander vermischt. Nach rund 10 Minuten wurde nach Gerinnung des Vollbluts eine gelartige Masse erhalten.

### BEISPIEL 10

Es wurde 5,0 g Granulat der Fraktion 1-3 mm des Beispiels 2 mit 2,5 g allogener Knochenchips (Größe der Chips 2-4 mm) miteinander vermischt. Es entstand ein homogenes Gemisch.

### BEISPIEL 11

Es wurde 5,0 g Granulat der Fraktion 1-3 mm des Beispiels 2 mit 15,0 g allogener Knochenchips (Größe der Chips 2-4 mm) miteinander vermischt. Es entstand ebenfalls ein homogenes Gemisch.

### BEISPIEL 12

Es wurde 5,0 g Granulat der Fraktion 1-3 mm des Beispiels 2 mit 5,0 g allogener Knochenchips (Größe der Chips 2-4 mm) und mit 5,0 ml (~ 5,0 g) frisches Vollblut (Schweineblut) miteinander vermischt. Es entstand ebenfalls ein homogenes Gemisch.

## Patentansprüche

1. Zusammensetzung zur Anwendung in einem Verfahren zur Behandlung eines Knochendefekts, wobei die Zusammensetzung Calciumsulfat, ein Erdalkalicarbonat, ein Bindemittel, und mehr als 0 bis höchstens 2,5 Gewichtsprozent Gentamicinsulfat, bevorzugt 0,5 bis 2,5 Gewichtsprozent Gentamicinsulfat, bezogen auf die Gesamtmasse der Zusammensetzung, umfasst.

2. Zusammensetzung zur Anwendung gemäß Anspruch 1, wobei die Anwendung ein Vermischen der Zusammensetzung mit lebensfähigen Zellen *in vitro* umfasst, wobei durch das Vermischen bevorzugt ein homogenes Gemisch gebildet wird.

3. Zusammensetzung zur Anwendung gemäß Anspruch 2, wobei die lebensfähigen Zellen in Form von Blut, bevorzugt Vollblut, gereinigten Knochenmarkzellen und/oder in Form von Spongiosa *in vitro* mit der Zusammensetzung vermischt werden.

4. Zusammensetzung zur Anwendung gemäß einem der vorangehenden Ansprüche, wobei die lebensfähigen Zellen autolog oder allogen sind.

5. Zusammensetzung zur Anwendung gemäß Anspruch 3 oder 4, wobei die Spongiosa in Form von Knochenchips vorliegt.

6. Zusammensetzung zur Anwendung gemäß Anspruch 5, wobei die Knochenchips einen mittleren Durchmesser von 1 bis 10 mm aufweisen.

7. Zusammensetzung zur Anwendung gemäß einem der Ansprüche 3 bis 6, wobei die Zusammensetzung *in vitro* in einem Gewichtsverhältnis von "1 zu 0,5" bis "1 zu 3" mit Spongiosa oder gereinigten Knochenmarkzellen vermischt wird.

8. Zusammensetzung zur Anwendung gemäß Anspruch 3, wobei die Zusammensetzung *in vitro* in einem Gewichtsverhältnis von "1 zu 0,2" bis "1 zu 1" mit Blut vermischt wird.

9. Zusammensetzung zur Anwendung gemäß einem der Ansprüche 3 bis 8, wobei jeweils ein Gewichtsteil der Zusammensetzung *in vitro* mit 0,5 bis 3,0 Gewichtsteilen Spongiosa oder gereinigten Knochenmarkzellen und mit 0,2 bis 0,8 Gewichtsteilen Blut vermischt wird.

10. Zusammensetzung zur Anwendung gemäß einem der Ansprüche 3 bis 9, wobei das Blut nach dem Vermischen mit der Zusammensetzung einer Gerinnung unterzogen wird.

11. Zusammensetzung zur Anwendung gemäß einem der vorangehenden Ansprüche, wobei die Behandlung das Einbringen der Zusammensetzung in einen Knochendefekt, beispielsweise eine Knochenkavität, umfasst.

12. Zusammensetzung zur Anwendung gemäß einem der vorangehenden Ansprüche, wobei die Zusammensetzung 70 bis 80 Gewichtsprozent Calciumsulfat umfasst.

13. Zusammensetzung zur Anwendung gemäß einem der vorangehenden Ansprüche, wobei die Zusammensetzung 12 bis 18 Gewichtsprozent Erdalkalicarbonat umfasst.

14. Zusammensetzung zur Anwendung gemäß einem der vorangehenden Ansprüche, wobei die Zusammensetzung 7 bis 12 Gewichtsprozent des Bindemittels umfasst.

15. Zusammensetzung zur Anwendung gemäß einem der vorangehenden Ansprüche, wobei der Knochendefekt ein traumatisch, onkologisch oder infektionsbedingt verursachter Knochendefekt ist.

16. Zusammensetzung zur Anwendung gemäß einem der vorangehenden Ansprüche, wobei die Zusammensetzung als Granulat vorliegt, wobei das Granulat Partikel mit einem mittleren Durchmesser von 1,0 bis 7,0 mm, beispielsweise 0,5 bis 10 mm, 1 bis 3 mm, 2 bis 6 mm, 3 bis 5 mm, oder 3,5 bis 4,5 mm, aufweist.

17. Zusammensetzung zur Anwendung gemäß Anspruch 16, wobei die Partikel eine unregelmäßige Form aufweisen, die durch Brechen eines Agglomerats herstellbar ist.
